(19) Europäisches Patentamt
European Patent Office
Office européen des brevets

(11) Publication number: **0 107 095**
**B1**

(12) **EUROPEAN PATENT SPECIFICATION**

(45) Date of publication of patent specification: **09.09.87**

(21) Application number: **83109686.2**

(22) Date of filing: **28.09.83**

(51) Int. Cl.[4]: **C 07 D 227/10,**
C 07 D 225/06,
C 07 D 223/16, A 61 K 31/55,
A 61 K 31/395

(54) **1-N-alkylcarboxy-benzofused lactams useful as antihypertensive agents.**

(30) Priority: **30.09.82 US 430918**

(43) Date of publication of application:
**02.05.84 Bulletin 84/18**

(45) Publication of the grant of the patent:
**09.09.87 Bulletin 87/37**

(84) Designated Contracting States:
**CH DE FR GB IT LI NL**

(56) References cited:
**EP-A-0 046 289**
**EP-A-0 046 291**
**EP-A-0 046 292**
**EP-A-0 072 352**

(73) Proprietor: **MERCK & CO. INC.**
**126, East Lincoln Avenue P.O. Box 2000**
**Rahway New Jersey 07065 (US)**

(72) Inventor: **Parsons, William H.**
**3004 Village Drive**
**Avenel New Jersey 07001 (US)**

(74) Representative: **Abitz, Walter, Dr.-Ing. et al**
**Abitz, Morf, Gritschneder, Freiherr von**
**Wittgenstein Postfach 86 01 09**
**D-8000 München 86 (DE)**

**EP 0 107 095 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

BACKGROUND OF THE INVENTION

1. Field of the Invention

The present invention is concerned with novel 1-N-alkylcarboxy-benzofused lactams which are effective inhibitors of angiotensin I converting enzyme. These novel compounds are, consequently, combined with pharmaceutically acceptable carriers to form pharmaceutical compositions of the present invention and are used in a method of treating hypertension.

Angiotensin II, a powerful vasoconstrictor hormonal peptide, is formed from the inactive angiotensin I by the action of angiotensin-converting enzyme. Recently, potent inhibitors of angiotensin-converting enzyme have been reported which are capable of lowering the blood pressure in hypertensive patients. The novel 1-N-alkylcarboxybenzofused lactams of the present invention are also potent inhibitors of angiotensin-converting enzyme.

2. Brief Description of the Prior Art

U.S. Patents 4,113,715; 4,129,571; and 4,154,960 disclose substituted acyl derivatives of amino acids which are useful as angiotensin converting enzyme inhibitors. More specifically, these compounds are mercapto substituted acyl amino acids and derivatives thereof including the clinically effective antihypertensive compound captopril, i.e., D-3-mercapto-2-methylpropanoyl-L-proline.

The foregoing prior art compounds are not dipeptide derivatives as are the compounds of the present invention. Furthermore, these prior art compounds contain an essential sulfhydryl substituent or derivative thereof whereas those of the present invention do not. In addition, the dipeptide compounds of the present invention are unusual dipeptides whose N-terminus bears a carboxymethyl group which is preferably further substituted on the methyl group. In addition, the carboxyl group(s) may also be converted to ester, amide and salt derivatives. In effect, the compounds of the present invention are hybrids formed by fusing α-amino acids onto dipeptides by means of a nitrogen shared by these two part-structures. This structural arrangement is rare in the field of synthetic and natural peptides and is not suggested or disclosed by the mercaptoacyl type functions of the two prior art patents identified above.

German Patent Application Nos. 2704—985 and 2720—996 describe hypotensive carboxy-acyl-proline derivatives and (N)-carboxyalkanoyl amino acid derivatives; and German Patent Application No. 2810—261 describes thio-substituted (N)-propionylamino acid derivatives which are hypotensive angiotensin converting enzyme inhibitors.

However, none of these references show or suggest any of the compounds of the present invention, which contain the $R^3$ functionality described herein.

An abstract of ACS meeting of 9-11-78 entitled "Superactive Analogs of the Angiotensin Converting Enzyme Inhibitor $BPP_{9a}$ Containing L-3,4-Dehydroproline" describes the use of dehydroproline on various peptides, none of which, however, are similar to the compounds of the present invention.

German Patent No. 30 46 271 A1 discloses optically active N-mercaptoalkanoylaminoacids which are useful as angiotensin converting enzyme inhibitors. Although this reference teaches that the claimed compounds have the same utility as those of the present invention, the compounds disclosed in the reference differ materially and significantly from those of the present invention especially since all of the reference compounds are mercapto-based.

Patchett et al., in "A New Class of Angiotensin-converting Enzyme Inhibitors", *Nature,* Vol. 288, No. 5788, pp. 280—283 (November 20, 1980), describe certain N-carboxymethyldipeptides. However, the compounds of the present invention are readily distinguishable by the presence of the $R^3$ functionality described herein.

EP—A—0,046,291, 0,046,292 and 0,072,352 disclose substituted caprolactam and enantholactam derivatives which are substituted by a carbonyl methyl group at the nitrogen of the lactam ring.

DETAILED DESCRIPTIONS OF THE INVENTION AND PREFERRED EMBODIMENTS

The present invention relates to novel 1-N-alkylcarboxybenzofused lactams of the formula:

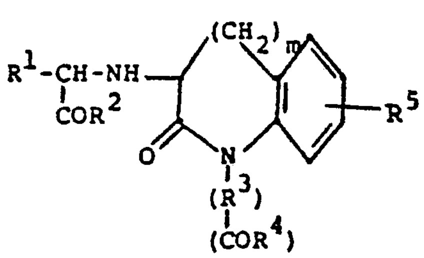

I

wherein

$R^1$ is alkyl or 1—10 carbon atoms which include branched, cyclic and unsaturated alkyl groups;

substituted $C_{1-6}$alkyl wherein the substituent can be hydroxy, $C_{1-6}$alkylthio, amino, $C_{1-6}$alkylamino, di-$C_{1-6}$alkylamino, $C_{1-6}$alkanoylamino, and benzoylamino; a group having the formula $R^1_A(CH_2)_n—Q—(CH_2)_p—$ wherein n is 0—2, p is 1—3, $R^1_A$ is phenyl, naphthyl, biphenyl or a 5- or 6-membered aromatic ring containing from one to three heteroatoms selected from the group consisting of nitrogen, oxygen and sulfur as well as any bicyclic group in which any of the above heterocyclic rings is fused to a benzene ring optionally substituted by $C_{1-6}$alkyl, halo, dihalo, amino, cyano, hydroxy, or $C_{1-6}$alkoxy, and Q is O, S, $N—R^1_B$, $CONR^1_C$, $NR^1_CCO$, or CH=CH wherein $R^1_B$ is hydrogen, $C_{1-6}$alkyl, phenyl, naphthyl, or biphenyl attached through a straight or branched chain hydrocarbon of from 1 to 6 carbon atoms, $C_{1-6}$alkanoyl, or benzoyl and $R^1_C$ is hydrogen or $C_{1-6}$alkyl; $C_{1-6}$alkyl substituted by phenyl, naphthyl, biphenyl or a 5- or 6-membered aromatic ring containing from one to three heteroatoms selected from the group consisting of nitrogen, oxygen and sulfur as well as any bicyclic group in which any of the above heterocyclic rings is fused to a benzene ring wherein the $C_{1-6}$alkyl portion can be substituted by amino, $C_{1-6}$alkanoylamino, benzoylamino or hydroxy and the aromatic ring portions can be substituted by $C_{1-6}$alkyl, halo, dihalo, amino, cyano, hydroxy, $C_{1-6}$alkoxy, amino-$C_{1-6}$alkyl, or hydroxy-$C_{1-6}$alkyl;

$R^2$ and $R^4$ are hydroxy; $C_{1-6}$alkoxy; phenyloxy, naphthyloxy, biphenyloxy; or $C_{1-6}$alkoxy substituted by phenyloxy, naphthyloxy, biphenyloxy;

m is 1 to 4;

$$R^3 \text{ is } -\!\left(\begin{matrix} R^a \\ | \\ C \\ | \\ R^b \end{matrix}\right)_{\!q}\!-$$

where

q is 2 or 3; provided that where q = 3, the carbon chain may be unsaturated between carbons 2—3;

the $R^a$'s and $R^b$'s are hydrogen; or $C_{1-6}$alkyl; and the $R^a$'s are additionally phenyl-$C_{1-6}$alkyl; amino-$C_{1-6}$alkyl; or $C_{1-6}$alkylthio-$C_{1-6}$alkyl; and

$R^5$ is hydrogen; or hydroxy;

and a pharmaceutically acceptable salt thereof.

The $C_{1-6}$alkyl substituents recited above represent, except where otherwise indicated, any of the variables of straight, branched, and unsaturated chain hydrocarbon radicals of from one to six carbon atoms, such as methyl, ethyl, *n*-propyl, *i*-propyl, *n*-butyl, *i*-butyl, *t*-butyl, *n*-pentyl, *i*-pentyl, *n*-hexyl; or vinyl, allyl, and butenyl.

The $C_{1-6}$alkoxy substituent represents a $C_{1-6}$alkyl group as described above attached through an oxygen bridge.

The aralkyl and heteroaralkyl substituents recited above represent, for example, benzyl, phenethyl, 3,3-diphenylpropyl, or 3-indolylmethyl.

Halo means chloro, bromo, iodo, or fluoro.

Examples of the above-mentioned 5- or 6-membered aromatic rings containing from one to three heteroatoms selected from nitrogen, oxygen, and sulfur are pyridyl, thienyl, furyl, imidazolyl, and thiazolyl; examples of bicyclic groups in which any of those heterocyclic rings is fused to a benzene ring are indolyl, quinolinyl, isoquinolinyl, benzimidazolylyl, benzothiazolyl, and benzthienyl.

The Formula I compounds can be used in the form of salts derived from inorganic or organic acids and bases. Included among such acid addition salts are the following: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfoate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nictotinate, oxalate, pamoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkane earth metal salts such as calcium and magnesium salts, salts with organic bases such as dicyclohexylamine and N-methyl-D-glucamine, and salts with amino acids such as arginine, lysine, and so forth. Water or oil-soluble or dispersible products are thereby obtained.

In the compounds of Formula I, the carbon atom to which $R^1$ is attached, as well as the carbon atoms of the $R^3$ substituent, may be asymmetric. These compounds accordingly exist in diastereo-isomeric forms or in mixtures thereof. Although the L- or S- configuration is preferred for each of the asymmetric carbon atoms, diastereomers containing D- or R- amino acids have activity dependent upon their structures and have advantages with respect to metabolic stability and can, therefore, be utilized in mixture or as pure diastereomeric compounds.

Preferred compounds of the present invention ar those of the Formula I wherein

m is 1 or 2,

the $R^a$'s and $R^b$'s are hydrogen; or $C_{1-6}$alkyl; and the $R^a$'s are additionally phenyl-$C_{1-6}$alkyl; amino-$C_{1-6}$alkyl; or $C_{1-6}$alkylthio-$C_{1-6}$alkyl; and

$R^5$ is hydrogen; or hydroxy.

Especially preferred compounds of the present invention are the following:

1-ethoxycarbonylethyl-3-(1-carboethoxy-3-phenyl-1-propyl)aminohomodihydrocarbostyril;
1-carboxyethyl-3-(1-carboxy-3-phenyl-1-propyl)aminohomodihydrostyril;
1-ethoxycarbonylethyl-3-(1-carboethoxy-3-phenyl-1-propyl)aminohexahydrobenzoazocine-2-one;
1-carboxyethyl-3-(1-carboxy-3-phenyl-1-propyl)aminohexahydrobenzoazocine-2-one, and ethyl diester;
1-(3-carboxypropyl)-3-(1-carboxy-3-phenyl-1-propyl)aminohomodihydrocarbostyril, and ethyl diester;
1-(4-carboxybutyl)-3-(1-carboxy-3-phenyl-1-propyl)aminohomodihydrocarbostyril, and ethyl diester;
1-carboxyethyl-3-(1-carboxy-3-phenyl-1-propyl)aminodihydrocarbostyril, and ethyl diester;
1-(3-carboxypropyl)-3-(1-carboxy-3-phenyl-1-propyl)aminohexahydrobenzoazocine-2-one, and ethyl diester;
1-(4-carboxybutyl)-3-(1-carboxy-3-phenyl-1-propyl)aminohexahydrobenzoazocine-2-one, and ethyl diester;
1-(3-carboxypropyl)-3-(1-carboxy-3-phenyl-1-propyl)aminodihydrocarbostyril, and ethyl diester;
1-(4-carboxybutyl)-3-(1-carboxy-3-phenyl-1-propyl)aminodihydrocarbostyril, and ethyl diester;
1-ethoxycarbonylethyl-3-(1-carboxy-3-phenyl-1-propyl)aminohomodihydrocarbostyril.

The compounds of this invention inhibit angiotensin converting enzyme and thus block conversion of the decapeptide angiotensin I to angiotensin II. Angiotensin II is a potent pressor substance. Thus blood-pressure lowering can result from inhibition of its biosynthesis especially in animals and humans whose hypertension is angiotensin II related. Furthermore, converting enzyme degrades the vasodilator peptide, bradykinin. Therefore, inhibitors of angiotensin converting enzyme may lower blood-pressure also by potentiation of bradykinin. Although the relative importance of these and other possible mechanisms remains to be established, inhibitors of angiotensin converting enzyme are effective antihypertensive agents in a variety of animal models and are useful clinically, for example, in many human patients with renovascular, malignant and essential hypertension. See, for example, D. W. Cushman et al., *Biochemistry 16,* 5484 (1977).

The evaluation of converting enzyme inhibitors is guided by *in vitro* enzyme inhibition assays. For example, a useful method is that of Y. Piquilloud, A. Reinharz and M. Roth, *Biochem. Biophys. Acta, 206,* 136 (1970) in which the hydrolysis of carbo-benzyloxyphenylalanylhistidinylleucine is measured. *In vivo* evaluations may be made, for example, in normotensive rats challenged with angiotensin I by the technique of J. R. Weeks and J. A. Jones, *Proc. Soc. Exp. Biol. Med., 104,* 646 (1960) or in a high renin rat model such as that of S. Koletsky et al., *Proc. Soc. Exp. Biol. Med., 125,* 96 (1967).

Thus, the compounds of the invention are useful in treating hypertension. They are also of value in the management of acute and chronic congestive heart failure, in the treatment of secondary hyper-aldosteronism, primary and secondary pulmonary hypertension, renal failure and renal vascular hypertension, and in the management of vascular disorders such as migraine or Raynaud's disease. The application of the compounds of this invention for these and similar disorders will be apparent to those skilled in the art.

Thus, in accordance with the present invention there is provided a pharmaceutical composition for treating hypertension comprising a pharmaceutically acceptable carrrier and an antihypertensively effective amount of a compound of Formula I.

For the purpose of treating hypertension and those clinical conditions noted above, the compounds of the present invention may be administered orally, topically, parenterally, by inhalation spray or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, dogs, cats, etc., the compounds of the invention are effective in the treatment of humans.

The pharmaceutical compositions containing the active ingredient may be in a form suitable for oral use, for example, as tablets, troches, lozenges, aqueous or oily suspensions, dispersible powders or granules, emulsions, hard or soft capsules, syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide a pharmaceutically elegant and palatable preparation. Tablets contain the active ingredient in admixture with non-toxic pharmaceutically acceptable excipients which are suitable for manufacture of tablets. These excipients may be, for example, inert diluents, such as calcium carbonate, sodium carbonate, lactose, calcium phosphate or sodium phosphate; granulating and disintegrating agents, for example maize starch, or alginic acid; binding agents, for example, starch, gelatine or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate alone or with a wax may be employed.

Formulations for oral use may also be presented as hard gelatine capsules wherein the active ingredient is mixed with an inert solid diluent, for example calcium carbonate, calcium phosphate or kaolin, or as soft gelatine capsules wherein the active ingredient is mixed with water or an oil medium, for

example arachis oil, peanut oil, liquid paraffin or olive oil.

Aqueous suspensions contain the active materials in admixture with excipients suitable for the manufacture of aqueous suspensions. Such excipients are suspending agents, for example sodium carboxymethyl cellulose, methylcellulose, hydroxypropylmethylcellulose, sodium alginate, polyvinylpyrrolidone, gum tragacanth and gum acacia; dispersing or wetting agents may be a natural-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyoxyethylene sorbitan mono-oleate. The said aqueous suspension, may also contain one or more preservatives, for example, ethyl or n-propyl p-hydroxy benzoate, one or more coloring agents, one or more flavoring agents and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oil suspensions may contain a thickening agent, for example beeswax, hard paraffin or cetyl alcohol. Sweetening agents, such as those set forth above, and flavoring agents may be added to provide a palatable oral preparation. These compositions may be preserved by the addition of an antioxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of oil-in-water emulsions. The oily phase may be a vegetable oil, for example olive oil or arachis oils, or a mineral oil, for example liquid paraffin or mixtures of these. Suitable emulsifying agents may be naturally-occurring gums, for example gum acacia or gum tragacanth, naturally-occurring phosphatides, for example soya bean lecithin, and esters or partial esters derived from fatty acids and hexitol anhydrides, for example sorbitan monooleate, and condensation products of the said partial esters with ethylene oxide, for example polyoxyethylene sorbitan mono-oleate. The emulsions may also contain sweetening and flavoring agents.

Syrups and elixirs may be formulated with sweetening agents, for example glycerol, sorbitol or sucrose. Such formulations may also contain a demulcent, a preservative and flavoring and coloring agents. The pharmaceutical compositions may be in the form of a sterile injectable preparation, for example as a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectibles.

The compounds of this invention may also be administered in the form of suppositories for rectal administration of the drug. These compositions can be prepared by mixing the drug with a suitable nonirritating excipient which is solid at ordinary temperatures by liquid at the rectal temperature and will theretofore melt in the rectum to release the drug. Such materials are cocoa butter and polyethylene glycols.

Dosage levels of the order of 70 to 3500 mg per patient per day, in single or multiple doses, are useful in the treatment of hypertension. Preferably, the dosage range will be from 350 to 2000 mg per day.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

The compounds of this invention can also be administered in combination with other antihypertensives and/or diuretics. For example, the compounds of this invention can be given in combination with such compounds as acetazolamide, amiloride, aminophylline, atenolol, bendroflumethiazide, benzthiazide, bumetanide, chlorothalidone, chlorothiazide, clonidine, cryptenamide acetates and cryptenamide tannates, cyclothiazide, deserpidine, diazoxide, diltiazem, (S)-1-[[2-(3,4-dimethoxyphenyl)-ethyl]amino]-3-[[4-(2-thienyl)-1H-imidazol-2-yl]phenoxy]-2-propanol, ethacrynic. acid, flumethiazide, furosemide, guanethidene sulfate, hydralazine hydrochloride, hydrochlorothiazaide, hydroflumethiazide, (+)-4-[3-[-[2-(1-hydroxycyclohexyl)ethyl]-4-oxo-2-thiazolidinyl]propyl]-benzoic acid, indacrinone and variable ratios of its enantiomers, merethoxylline procaine, methylclothiazide, methyldopa, methyldopate

hydrochloride, metolazone, metoprolol tartate, minoxidil, naldolol, nifedipine, pargyline hydrochloride, pindolol, polythiazide, prazosin, propranolol, quinethazone, *rauwolfia serpentina,* rescinnamine, reserpine, sodium ethacrynate, sodium nitroprusside, spironolactone, ticrynafen, timolol, triamterene, trichloromethiazide, trimethophan camsylate, verapamil, and the like, as well as admixtures and combinations thereof.

Typically, the individual daily dosages for these combinations can range from about one-fifth of the minimally recommended clinical dosages to the maximum recommended levels for the entities when they are given singly.

To illustrate these combinations, one of the antihypertensives of this invention effective in the 350 to 2000 mg per day range can be effectively combined at levels at the 70 to 2000 mg per day range with the following compounds at the indicated per day dose range: hydrochlorothiazide (10—100 mg); chlorothiazide (125—2000 mg); manipulated indacrinone enantiomer ratio (25—150 mg); ethacrynic acid (15—2000 mg); amiloride (5—20 mg); furosemide (5—80 mg); propranolol (20—480 mg); ; timolol (5—60 mg); and methyldopa (65—2000 mg); and the pivaloyloxyethyl ester of methyldopa (30—1000 mg). In addition, triple drug combinations of hydrochlorothiazide (10—100 mg) plus amiloride (5—20 mg) plus converting enzyme inhibitor of this invention (70—2000 mg); hydrochlorothiazide (10—100 mg) plus timolol (5—60 mg) plus the converting enzyme inhibitor of this invention (70—2000 mg); or manipulated indacrinone enantiomer ratio (25—150 mg) plus amiloride (5—20 mg) plus converting enzyme inhibitor of this invention (70—2000 mg) are effective combinations to control blood pressure in hypertensive patients. Naturally, these dose ranges can be adjusted on a unit basis as necessary to permit divided daily dosage and, as noted above, the dose will vary depending on the nature and severity of the disease, weight of patient, special diets and other factors.

Typically, these combinations can be formulated into pharmaceutical compositions as discussed below.

Dosage levels of the order of 20 to 100 mg per kg, or 1400 to 7000 mg per patient per day, in single or multiple doses, are useful in the treatment of pain. Preferably, the dosage range will be from 2000 to 5000 mg per day.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

About 70 to 2000 mg of a compound or mixture of compounds of Formula I or a physiologically acceptable salt is compounded with a physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

The compounds of Formula I can be prepared by one or more of the methods described further below.

As will be evident to those skilled in the art and as demonstrated in the Examples which follow, reactive groups not involved in the reaction, such as amino, carboxy, mercapto, etc., may be protected by methods standard in peptide chemistry prior to the coupling reactions and subsequently deprotected to obtain the desired products.

The compounds of formula I may be prepared by any convenient process. Useful processes are illustrated by the following reaction equations.

Process A

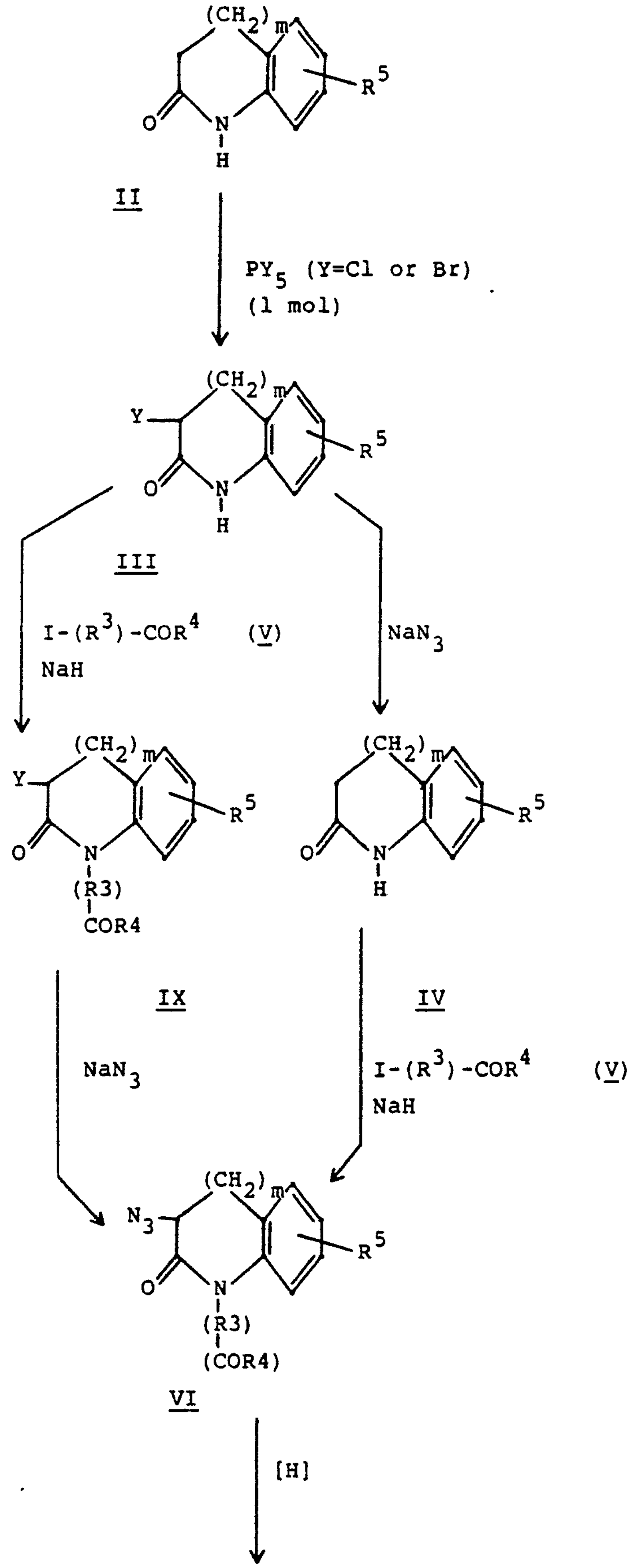
II
PY$_5$ (Y=Cl or Br)
(1 mol)
III
I-(R$^3$)-COR$^4$ (V)
NaH
NaN$_3$
IX
IV
NaN$_3$
I-(R$^3$)-COR$^4$ (V)
NaH
VI
[H]

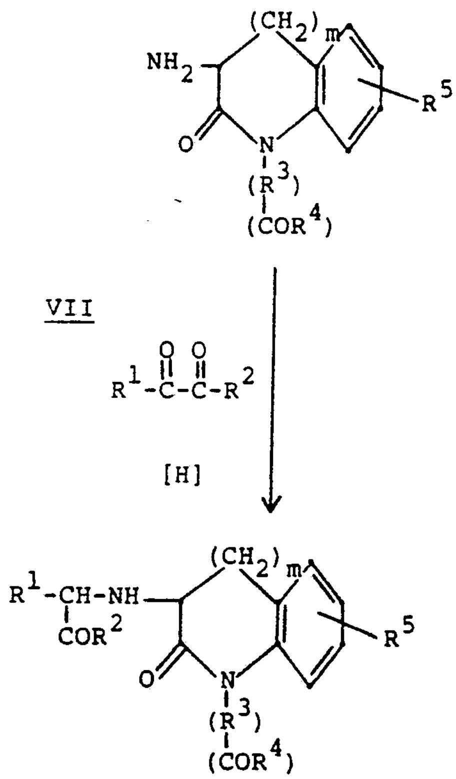


I' (I'=Formula I where $R^2$=$R^4$=OH)

Alternate Process for IX

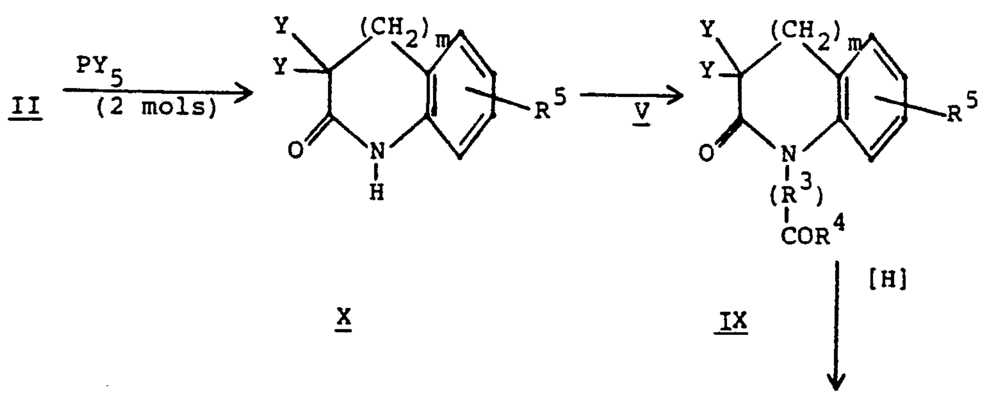


X

IX

Process B

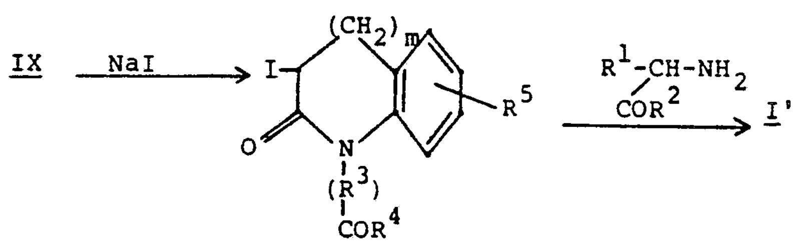

Process C

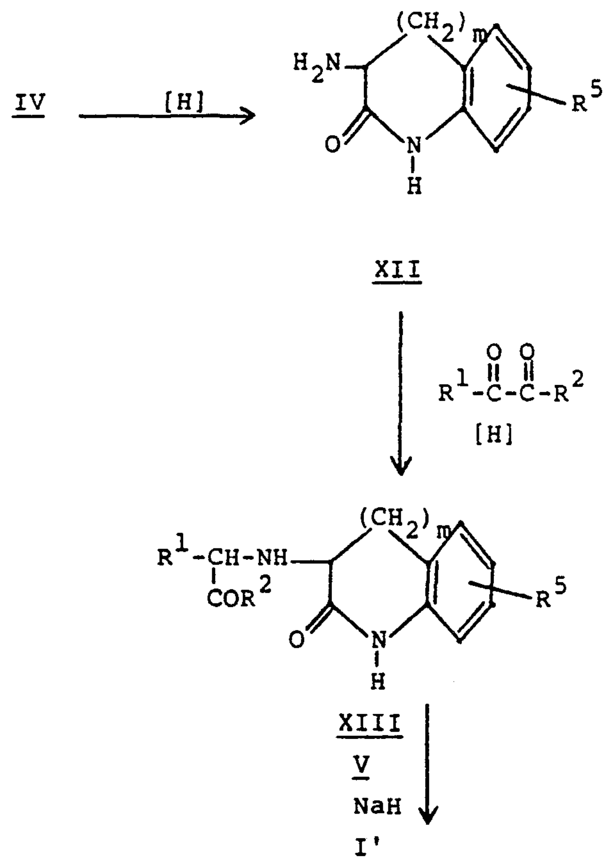


*Process A*

Benzofused lactam (II) ring size ranging from 6 to 9 (m = 1—4), prepared from a precursor ketone by a procedure of Blicke *et al., J. Am. Chem. Soc., 76,* 2317 (1954), is converted to (III), with $PX_5$ where X = Br or Cl [Nagasawa *et al., J. Med. Chem., 14,* 501 (1971)]. Reaction of (III) with sodium or lithium azide in a suitable solvent such as DMF or ethanol [see, for example, Brenner *et al., Helv. Chem. Acta, 41,* 181 (1958)] affords (IV) which can be alkylated with an iodoester (V) in the presence of a strong base, like sodium hydride, in a solvent such as DMF or THF to produce (VI). Reduction of (VI) with hydrogen and a suitable catalyst, such as palladium on carbon, affords (VII). Intermediate (VII) is then reductively coupled with a keto acid or ester (VIII) in a solvent such as ethanol using a catalyst such as palladium on carbon to afford (I') ($R_5$ and $R_6 \neq$ H). Alternatively, sodium cyanoborohydride can be used to effect the reduction.

Groups $R^2$ and $R^4$ may be modified by known methods, if desired. For example, if $R^4$ = Et and $R^2$ = t-Bu, the diester (I) can be converted to the monoester $R^4$ = Et and $R^2$ = H by treatment with trifluoroacetic acid. If $R^4 = R^2$ = Et, (I) can be converted to the diacid $R^4 = R^2$ = H by basic hydrolysis.

Alternatively, (III) may be alkylated with (V) in the presence of a strong base, like sodium hydride, and the intermediate (IX) converted to (VI) by reaction with an azide salt as described above.

In the above preparation, the keto acid or ester represented by the formula

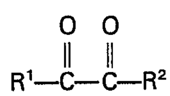

may be, for example, 2-oxo-4-phenyl-butyric acid. Other α-keto acids or esters may be utilized to prepare other compounds of the present invention for various definitions of $R^1$ and $R^2$. Such α-keto acids are readily available or may be prepared by well-known techniques. For example, synthons such as

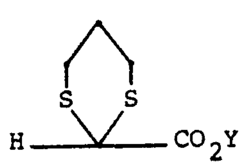

can be converted to α-keto acids or esters using methods involving alkylation followed by hydrolysis as described in the literature. An excellent method involves the reaction of Grignard reagents $R_1MgX$ with $ClCOCO_2Y$ or $YO_2CCO_2Y$. Another method involves condensing substituted acetic acid esters with diethyl oxalate followed by hydrolytic decarboxylation under acidic conditions to obtain α-keto acids. Carefully controlled acid hydrolysis in alcohol of acyl cyanides, which are prepared from acid chlorides and cuprous cyanide, also proves to be a viable synthetic route to α-keto esters. Nucleophilic displacement reactions on chloro or bromo pyruvic acid (ester) can also be used to produce a variety of interesting α-keto acids (esters). In these formulae, Y is a group such as loweralkyl or benzyl and protecting groups are employed as necessary in the $R^1$ group if an interfering functionality is present.

Additional compounds of Formula I can be prepared by employing the keto acids and esters listed in Table I below.

TABLE I

Keto Acids and Esters of the Formula $R^1$-C=O
COOR (II.)

(a) 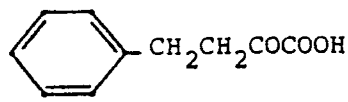

(b) 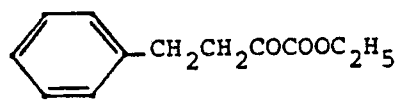

(c) 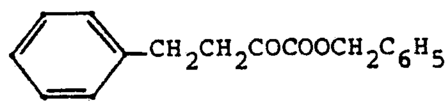

(d) 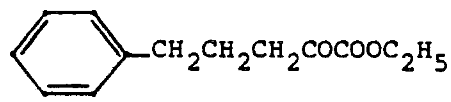

(e) 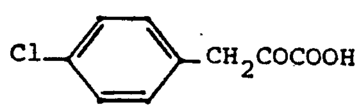

(f) 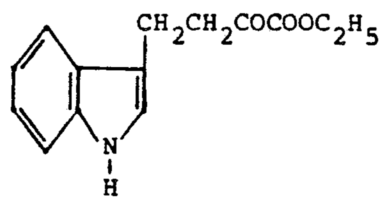

(g) 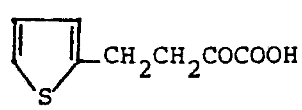

(h) 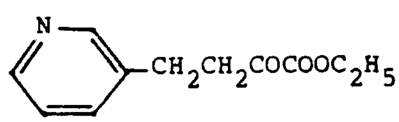

(i) 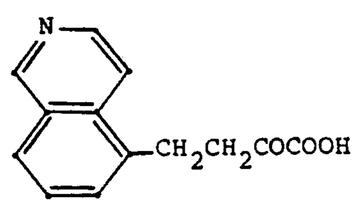

TABLE I

Keto Acids and Esters of the Formula 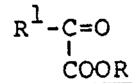 (II.)

(j) 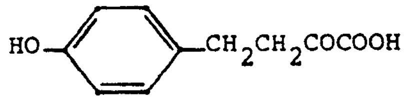

(k) 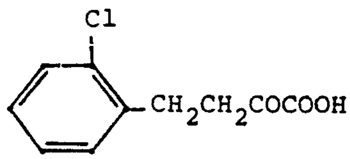

(l) 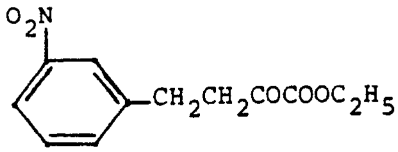

(precursor for the corresponding amino compound)

(m) 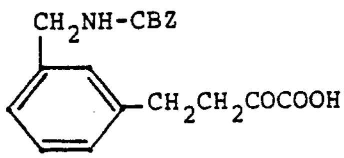

(precursor for the corresponding amino compound)

(n) 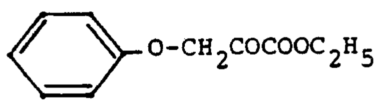

(o) 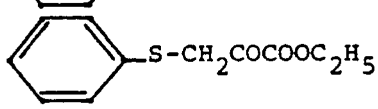

(p) $CH_3S-CH_2CH_2COCOOH$

(q) $(CH_3)_2-CH_2CH_2COCOOH$

(r) $CBZ-HN(CH_2)_4-COCOOH$

(precursor for the corresponding amino compound)

(s) 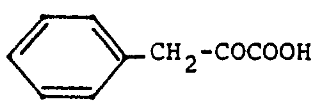

(t) 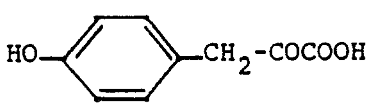

(u) 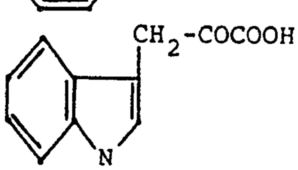

*Alternate Process for IX*

If desired, (IX) may be prepared by the alkylation of (X) [prepared from II using the alternate conditions of Nagasawa, above] with (V) to afford intermediate (XI). Treatment of (XI) with hydrogen and a catalyst, such as palladium on carbon, affords (IX).

*Process B*

Alternatively, (IX) (X = Cl, Br; $R^4 \neq H$) may be converted to the iodo compound (IX) (X = I) by known methods, for example, sodium iodide in acetone. Reaction of this iodo lactam with an amino acid ester in a solvent such as toluene or DMF in the presence of a halide scavenger such as $Ag_2O$ gives (I) ($R^2 \neq H$, $R^4 \neq H$).

*Process C*

If desired, IV may be reduced with hydrogen in the presence of a suitable catalyst to afford (XII) which can be alkylated with a ketoester in the presence of hydrogen on a suitable catalyst to give (XIII). Alternatively, sodium cyanoborohydride may be used. Alkylation of (XIII) to afford (I') ($R^2 \neq H$, $R^4 \neq H$) can be carried out with an iodoester in the presence of a strong base such as sodium hydride in a solvent such as THF.

In the compound of Formula I, the carbon atom to which $R^1$ is attached, the carbon atoms of $R^3$, and the ring carbon atom to which the

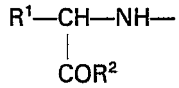

group is attached may be asymmetric. Thus, the compounds of this invention exist in diastereoisomeric forms or in mixtures thereof. The process described above can utilize racemates, enantiomers or diastereomers as starting materials. When diastereomeric intermediates or products result from the synthetic procedures, the diastereomeric intermediates or products can be separated by chromatographic or fractional crystallization methods. When racemic mixtures result, they may be resolved by crystallization of salts of optically active acids or bases or by other methods known in the art. The part-structures

$—N—(R^3)—COR^4$ 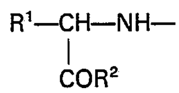 and 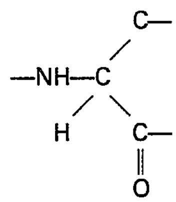

of Formula I can be in two configurations (S or R) and both are within the scope of this invention, although S is generally preferred.

The following Examples illustrate preparation of representative compounds of Formula I. All temperatures are in °C.

### Example 1

A. 3-Bromo-homodihydrocarbostyril

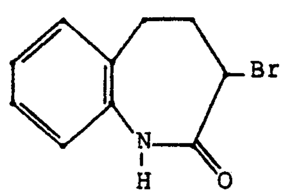

A

To a solution of 15 gm (0.093 mol) of homo dihydrocarbostyril (L. H. Briggs, J.C.S., 456 (1937)) in 200 ml of chloroform was added in increments over a period of an hour 19 gm of $PCl_5$ at which time was added 140 mg of iodine followed by a slow dropwise addition of 90 ml of a 1M solution of bromine in chloroform. The reaction mixture was warmed to room temperature where stirring was continued a further 1 hour.

The crude reaction mixture was concentrated *in vacuo* and then partitioned between water, ice and chloroform. The aqueous layer was extracted 2 times with methylene chloride and the combined organic fractions were filtered through $MgSO_4$ and concentrated *in vacuo*. The crude bromide *D* was chromatographed (silica, 2:1 ether:hexanes) to give 6.5 gm of pure *A* bromide.

TLC (silica, 2:1 ether:hexanes) $R_f$ = 0.65.
NMR ($CDCl_3$, TMS) 2.4—3.0 (m, 4H); 4.4—4.7 t, 1H); 7.2 (s, 4H); 9.2 (bs, 1H).
IR 1650 $cm^{-1}$.
mass spectrum: $M^+$ 239, m/e: 241 ($M^+$, +2); 160 ($M^{+2}$ −Br); 132 (—CO=O).

B. 3-Azido homodihydrocarbostyril

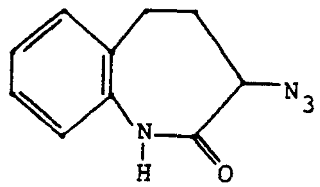

B

To a solution of 9.98 gm (0.0417 mol) of 3-bromo homodihydrocarbostyril in 200 ml of dimethylformamide (DMF) was added 10.8 gm of sodium azide stirring 12 hours at 60°C at which time the DMF was removed at reduced pressure. To the crude reaction mixture was added 50 ml of water and the mixture was then extracted 3 times with 50 ml of chloroform. The combined organic fractions were combined, washed with 25 ml of a saturated solution of NaCl, filtered through $MgSO_4$ and concentrated at reduced pressure. Chromatography (silica, 2:1 ether:hexanes) gave 7.92 gm of pure *B* azide. m.p. 150—151°C.

TLC (silica, 2:1 ether:hexanes) $R_f$ = 0.71.
El. An. Calc. for $C_{10}H_{10}N_4O$: N, 27.71; C, 59.39; H, 4.98
Found: N, 27.27; C, 59.25; H, 4.98
NMR ($CDCl_3$, TMS) 2.2—2.8 (m, 4H), 3.6—4.0 (dd, 1H); 7.2 (bs, 4H) 9.2 (bs, 1H) IR $N_3$ 2130, CO 1678.
mass spectrum: $M^+$ 202, m/e: 174 ($M^+$ $-N_2$; 146 (C = 0).

C. 1-Ethoxycarbonylethyl-3-azidohomodihydrocarbostyril

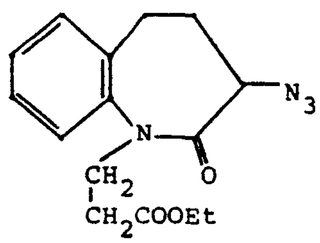

C

To a suspension of 0.476 gm of sodium hydride (50% dispersion in oil washed 3 times with hexanes) in 10 ml of tetrahydrofuran (THF) at 0°C was added dropwise a solution of 2.0 gm (0.01 mol) of 3-azido homodihydrocarbostyril and 1.32 ml of ethyl 3-iodopropionate in 20 ml of THF. The reaction was carefully monitored by TLC (silica, 1:1 ether:hexanes) until reaction was complete at which time the reaction was quenched with 30 ml of saturated $NH_4Cl$, diluted with 20 ml $H_2O$ and extractd 3 times with 50 ml $CH_2Cl_2$. The combined organic layers were filtered, concentrated *in vacuo* and chromatographed (silica, 1:1 ether:hexanes) to give 1.56 gm of pure product *C* as an oil.

TLC (silica, 1:1 ether:hexanes) $R_f$ = 0.375.
NMR ($CDCl_3$, TMS) 1.08 (+, 3H), 2.1—2.9 (m, 6H), 3.5—4.8 (m, 5H), 7.2 (bs, 4H).
El. An. Calc. for $C_{15}H_{18}N_4O_3$: C, 59.59; H, 6.00; N, 18.53
Found: C, 59.83; H, 6.13; N, 18.55

D. Ethoxycarbonylethyl-3-aminohomodihydrocarbostyril

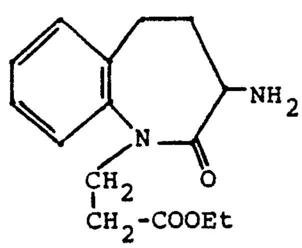

D

A solution of 2 gm of (0.00662 mol) of 1-ethoxycarbonylethyl-3-azidohomodihydrocarbostyril in 25 ml

of absolute ethanol with 0.2 gm of Pd/C 10% was hydrogenated for 12 hr at room temperature and 40 lbs of $H_2$. The reaction was subsequently filtered and the ethanol removed at reduced pressure to give 1.8 gm of pure amine *D*.

NMR ($CDCl_3$, TMS) 1.2 (+, 3H) 1.8—3.0 (m, 8H), 3.2—3.5 (m, 1H), 4.0 (q, 2H), 4.3—4.8 (m, 2H), 7.2 (s, 4H).

E. · 1-Ethoxycarbonylethyl-3-(1-carboethoxy-3-phenyl-1-propyl)-aminohomodihydrocarbostyril (racemate mixture)

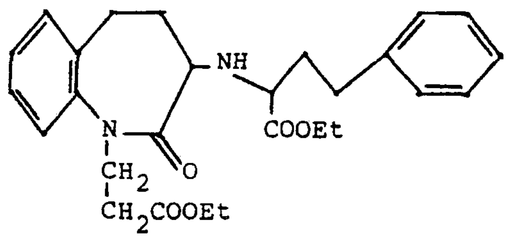

E

A solution of 2.4 gm (0.0087 mol) of ethoxycarbonylethyl-3-aminohomodihydrocarbostyril, 6.27 gm of ethyl 4-phenyl-2-oxobutyrate and 0.52 gm of acetic acid in 12 ml of absolute ethanol was stirred 1 hour at room temperature. To the stirred solution was added dropwise over a 12 hour period a solution of 1.37 gm of $NaCNBH_3$ in 12 ml of ethanol. After stirring a further 8 hours the reaction was concentrated at reduced pressure and partitioned between $H_2O$ and ethyl acetate. After extracting twice with ethyl acetate the combined organic layers were filtered through $MgSO_4$ and concentrated *in vacuo*. The crude reaction mixture was chromatographed (silica, 1:1 ethyl acetate: hexanes) and two diastereomeric *E* racemates were isolated as oils.

*Racemate Diester A (620 mg):*

TLC (silica, 1:1, hexanes:ethyl acetate) $R_f$ = 0.69.

NMR ($CDCl_3$, TMS) 1.08 (t, 3H), 1.28 (t, 3H), 1.7—3.2 (m, 10H), 3.6—4.6 (m, 8H), 7.1 (s, 4H), 7.2 (s, 5H).

El. An. Calc. for $C_{27}H_{37}O_5N_2$: C, 69.50; H, 7.35; N, 6.00.
Found: C, 69.70; H, 7.45; N, 5.78

*Racemate Diester B (310 mg):*

TLC (silica, 1:1, ethyl acetate:hexanes) $R_f$ = 0.61

NMR ($CDCl_3$, TMS) 1.2 (2 overlapping t, 6H), 1.7—2.9 (m, 11H), 3.0—3.3 (m, 2H), 3.5—4.7 (m, 6H) 7.1 (s, 9H).

El. An. Calc. for $C_{27}H_{37}N_2O_5$: C, 69.50; H, 7.35; N, 6.00
Found: C, 69.72; H, 7.35; N, 5.96

Example 2

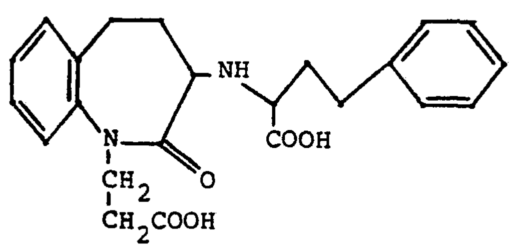

E

1-Carboxyethyl-3-(1-carboxy-3-phenyl-1-propyl)aminohomodihydrostyril

*Racemate A*

To a solution of 0.45 gm (0.00094 mol) of racemate diester *A* of Example 1 was added 1.8 ml of a 2N NaOH in water solution. The reaction mixture was stirred 8 hours at which time the reaction mixture was partially concentrated *in vacuo* and made acidic with acetic acid. The reaction mixture was partitioned between chloroform and water. After extracting twice with chloroform the combined organic fractions were filtered through $Na_2SO_4$ and concentrated *in vacuo*. Upon trituration with ether 200 mg of pure diacid crystallized out and were recovered by filtration.

NMR ($CD_3OD$, $ND_3$) (2.0—3.1) (m, 10H), 3.7—4.5 (m, 4H) 7.2—7.4 (2 overlapping s, 9H). M.P. 139—140°C.

El. An. Calc. for $C_{23}H_{26}H_2O_5 . 1.5\ H_2O$: C, 63.09; N, 6.30; H, 6.62.
Found: C, 63.36; N, 5.92; H, 6.33.

*Racemate B*

310 mg of racemate diester *B* of Example 1 were converted to the diacid by the same procedure described above to give 1.75 mg of free diacid. M.P. 222—225°C.

NMR ($CD_3OH$, $ND_3$) 2.2—3.4 (m, 10H), 3.5—4.4 (m, 4H), 7.2—7.5 (m, 9H).

| | | | |
|---|---|---|---|
| El. An. Calc. for $C_{23}H_{26}N_2O_5$.1.25 $H_2O$: | C, 63.74; | N, 6.46; | H, 6.46. |
| Found: | C, 63.96; | N, 6.37; | H, 6.16. |

Example 3

A. 3-Bromohexahydrobenzoazocin-2-one

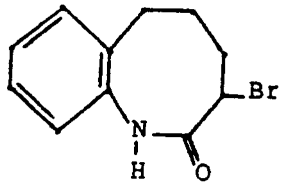

F

To a solution of 15 gm (0.084 mol) of hexahydrobenzoazocin-2-one (Chemica Scandinavia, *18*, 191 (1964) in 150 ml of chloroform at 0°C was added 8.8 gm of $PCl_5$ in increments over a period of an hour. Subsequently, to the reaction mixture was added 70 mg of iodine followed by slow dropwise addition of 84 ml of a 1M solution of bromine in chloroform. The reaction mixture was warmed to room temperature and stirred for 1 hour at which time it was concentrated at reduced pressure. To the crude product was added a mixture of ice and water and the mixture was extracted 3 times with methylene chloride. The combined organic fractions were filtered through $MgSO_4$ and concentrated at reduced pressure. The solid bromide was recrystallized with a mixture of chloroform and hexanes to give 12 gm of pure product *F*. M.P. 194—195°C.

TLC (silica, 2:1 ether:hexanes) $R_f = 0.36$

| | | | |
|---|---|---|---|
| An. Calc. for $C_{11}H_{12}NOBr$.1/4$H_2O$: | N, 5.41; | C, 51.08; | H, 4.68 |
| Found: | N, 5.29; | C, 50.75; | H, 4.53 |

NMR ($CDCl_3$, TMS) 1.6—2.9 (m, 6H); 4.2—4.5 (bt, 1H); 7.2 (s, 4H); 8.5 (bs, 1H);

Mass spectrum: $M^+$ 253, m/e: 252 ($p^+2$, 10%); 179 ($M^+$-Br); 146 (C=O).

B. 3-Azidodexahydrobenzoazocine-2-one

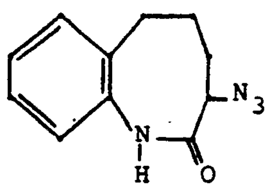

G

To a solution of 10 gm (0.00394 mol) of 3-bromohexahydrobenzoazocine-2-one in 100 ml of dimethylformamide was added 10 gm of sodium azide and the resultant reaction mixture was stirred 12 hours at 60°C.

The DMF was then removed at reduced pressure and the crude product was partitioned between $H_2O$ and methylene chloride. The aqueous layer was extracted 3 times with methylene chloride and the combined organic fractions were filtered through $MgSO_4$ and concentrated at reduced pressure. The product was chromatographed (silica, 2:1 ether:hexanes) giving 8 gm of pure azide *G*. M.P. 142—143°C.

TLC (silica, 2:1 ether:hexanes) $R_f = 0.45$

| | | | |
|---|---|---|---|
| An. Calc. for $C_{11}H_{12}N_4O$.1/4$H_2O$: | N, 36; | C, 59.79; | H, 5.44 |
| Found: | N, 25.17; | C, 59.37; | H, 5.39 |

NMR ($CDCl_3$, TMS) 1.6—2.9 (m, 6H); 3.4—3.7 (bt, 1H); 7.2 (s, 4H); 8.5 (bs, 1H);

Mass spectrum: m/e 188, ($M^+$-$N_2$); 159 (C=O).

C. 3-Azido-1-ethoxycarbonylmethyl-hexahydrobenzoazocine-2-one

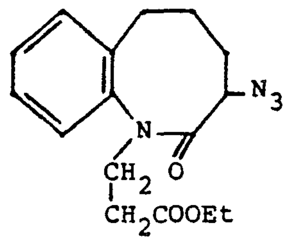

H

To a suspension of 0.655 gm of sodium hydride (50% suspension, prewashed with hexanes) in 15 ml THF at 0°C was added dropwise a solution of 3 gm (0.013 mol) of azide lactam and 1.74 ml of ethyl 3-iodopropionate in 15 ml of THF. The reaction was found to be complete upon the completion of the addition. (TLC, silica gel, 2:1 ether:hexanes). The reacton mixture was then quenched by the addition of 20 ml of saturated $NH_4Cl$. The solution was extracted three times with 50 ml portions of ethyl acetate. The combined organic fractions were filterd through $MgSO_4$ and concentrated *in vacuo*. The crude product was purified by chromagraphy on silica gel using 1:1 ether:hexanes as eluant. The fractions containing the desired product were combined and concentrated at reduced pressure to give 2.8 gm of the desired product *H*. TLC (7:3, hexanes:ethyl acetate, silica) $R_f$ = 0.67. M.P. 72—74°C.

NMR ($CDCl_3$, TMS) 1.1 (t, 3H), 1.8—3.0 (m, 8H) 3.2—4.6 (m, q, m, 5H), 7.2 (s, 4H).

D. 3-Amino-1-ethoxycarbonylethyl-hexahydrobenzoazocine-2-one

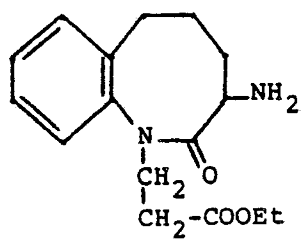

I

A solution of 4.5 gm (0.00142 mol) of azide lactam *H* in 50 ml of absolute ethanol with 450 mg of 10% Pd/C was hydrogenated 12 hours at 40 lbs of $H_2$ at room temperature. The reaction mixture was subsequently filtered and the filtrate concentrated *in vacuo* to yield 4.3 gm of amine *I* as an oil.

NMR ($CDCl_3$, TMS) 1.15 (t, 3H), 1.4—3.1 (m, 10H) 3.4—4.5 (m, 5H), 7.2 (s, 4H).

E. 1-Ethoxycarbonylethyl-3-(1-carboethoxy-3-phenyl-1-propyl)aminohexahydrobenzoazocine-2-one (racemate mixture)

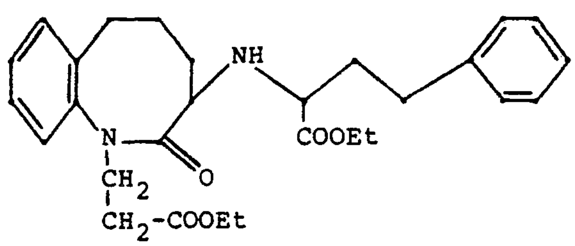

4 gm of amino ester *I* was reductively alkylated with 11 gm of ethyl 4-phenyl-2-oxobutyrate by the same procedure as in Example 1, Step E. The crude reaction product mixture was chromatographed (silica, 1:1 ethyl acetate:hexanes) and two diasteriomeric racemates were isolated as oils.

Racemate Diester A (1.3 gm):

TLC (silica, 1:1 hexanes:ethyl acetate) $R_f$ = 0.67.

NMR ($CDCl_3$, TMS) 1.08 (t, 3H), 1.12 (t, 3H), 1.5—3.1 (m, 11H), 3.5—4.6 (m, 8H), 7.0—7.3 (m, 9H).

| | | | |
|---|---|---|---|
| El. An. Calc. for $C_{28}H_{36}$—$H_2O_5$: | N, 5.83; | C, 69.97; | H, 7.55; |
| Found: | N, 5.70; | C, 69.99; | H, 7.62. |

Racemate Diester B (1.6 gm):

TLC (silica, 1:1 ether:hexanes) $R_f = 0.54$.

NMR ($CDCl_3$, TMS) 1.04 (t, 3H), 1.07 (t, 3H) 1.62—2.8 (m, 9H), 2.9—3.2 (m, 2H), 3.5—4.55 (m, 8H), 7.1 (bs, 9H)].

El. An. Calc. for $C_{28}H_{36}N_2O_5$: N, 5.83; C, 69.97; H, 7.55
Found: N, 5.69; C, 69.88; H, 7.66.

Example 4

1-Carboxyethyl-3-(1-carboxy-3-phenyl-1-propyl)aminohexahydrobenzoazocine-2-one

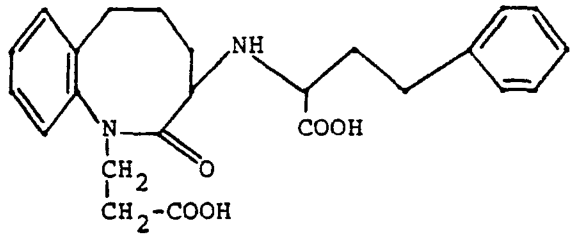

K

Racemate A

The racemate A diester of Example 3, (600 mg, 0.00122 mol) was converted to the diacid by the same procedure as in example 2 to give 350 mg of diacid racemate A.

NMR ($D_2O$, $ND_3$) 1.7—3.0 (m, 12H), 3.2—3.5 (bt, 2H), 3.7—4.4 (m, 2H), 6.9—7.3 (m, 9H).

Racemate B

By the same procedure 600 mg of diester racemate B was converted to 230 mg of the racemate diacid B.

NMR ($D_2O$, $ND_3$) 1.6—3.3 (m, 14H), 3.6—4.5 (m, 2H) 7.3—7.5 (m, 9H).

Example 5

A. 1-(3-Ethoxycarbonylpropyl)-3-azidohomodihydrocarbostryil

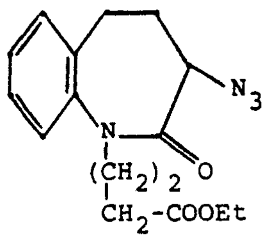

L

By the same alkylation procedure as Example 1, Step C, 4 gm of 3 azidohomodihydrocarbostyril was alkylated with ethyl 4-iodobutyrate to give upon chromatography (silica, 7:32, hexanes:ethyl acetate) 2.4 gm of pure product *L* as an oil.

TLC (silica, 2:1 hexanes:ethyl acetate) $R_f = 0.58$.

NMR ($CDCl_3$, TMS) 1.2 (t, 3H), 1.7—2.9 (m, 8H), 3.3—4.5 (m, 5H), 7.1 (bs, 4H).

El. An. Calc. for $C_{16}H_{20}N_4O_3$: N, 17.71; C, 60.75; H, 6.37
Found: N, 17.66; C, 60.92; H, 6.32.

B. 1-(3-Ethoxycarbonylpropyl)-3-(1-carboethoxy-3-phenyl-1-propyl)aminohomodihydrocarbostyril (racemate mixture)

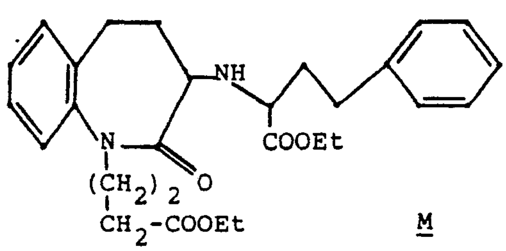

M

By the same sequence of procedures as Example 1, Steps D and E, 1.25 gm (0.004 mol) of the azide

ester prepared above was converted to the free amino ester which was subsequently reductively alkylated with ethyl 4-phenyl-2-oxobutyrate and the product mixture chromatographed (silica, 1:1 hexanes:ethyl acetate) to give the diasteriomeric racemates as oils.

Racemate A (370 mg):

TLC (silica, 2:1 hexanes:ethyl acetate) $R_f = 0.69$.

NMR ($CDCl_3$, TMS) 1.2 (2 overlapping t, 6H), 1.7—3.8 (m, 17H), 1.9—4.4 (2 overlapping q, 4H), 7.1—7.3 (2 overlapping s, 9H).

| | | | |
|---|---|---|---|
| El. An. Calc. for $C_{28}H_{36}N_2O_5$: | N, 5.83; | C, 69.97; | H, 7.55 |
| Found: | N, 5.59; | C, 69.88; | H, 7.22 |

Racemate B (410 mg):

TLC (silica, 2:1 hexanes:ethyl acetate) $R_f = 0.61$.

NMR ($CDCl_3$, TMS) 1.1 (t, 3H), 1.2 (t, 3H), 1.7—3.6 (m, 17H), 3.9—4.3 (overlapping q, 4H), 7.2 (s, 9H).

| | | | |
|---|---|---|---|
| El. An. Calc. for $C_{28}H_{36}N_2O_5$: | N, 5.83; | C, 69.97; | H, 7.55 |
| Found: | N, 5.73; | C, 69.57; | H, 7.56 |

C. 1-(3-carboxypropyl)-3-(1-carboxy-3-phenyl-1-propyl)aminohomodihydrocarbostyril

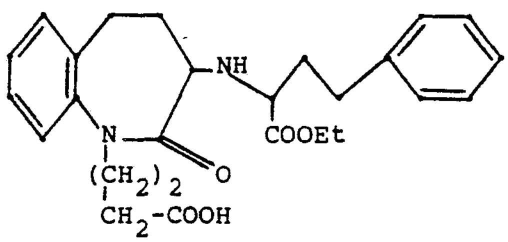

N

Racemate B

480 mg of diester racemate B was converted to the diacid by a procedure identical to Example 2.

Diacid racement B (270 mg):

NMR ($CD_3OD$) 1.7—3.0 (m, 12H), 3.4—4.5 (m, 4H), 7.1—7.2 (overlapping s, 9H).

| | | | |
|---|---|---|---|
| El. An. Calc. for $C_{24}H_{28}H_2O_5.1H_2O$: | N, 6.32; | C, 65.08; | H, 6.37; |
| Found: | N, 6.10; | C, 64.76; | H, 6.15 |

Example 6

A. 1-(4-Ethoxycarbonylbutyl)-3-azidohomodihydrocarbostyril

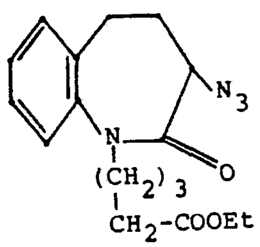

O

Using the procedures of Example 1, Step C, 1.46 gm of azide ester were prepared from 3-azidohomodihydrocarbostyril by alkylation with ethyl 4-iodopentanoate. The product was isolated as an oil.

NMR ($CDCl_3$, TMS) 1.2 (t, 3H), 1.4—1.8 (m, 4H), 2.0—3.1 (m, 6H), 3.3—3.8 (m, 2H), 3.9—4.4 (q+u, 8H) 7.2 (s, 4H).

B. 1-(4-Ethoxycarbonylbutyl)-3-(1-carboethoxy-3-phenyl-1-propyl)aminohomodihydrocarbostyril (racemate mixture)

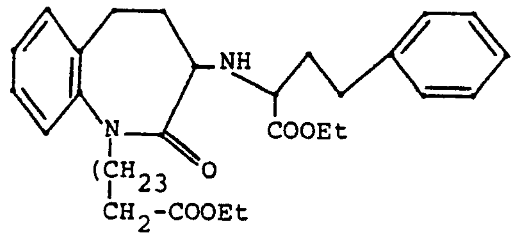

P

By the same sequence of procedures as Example 1, Steps D and E, 1.46 gm of azide ester was first catalytically reduced and then reductively alkylated with ethyl 4-phenyl-2-oxobutyrate and the product mixture chromatographed (silica, 1:1 ethyl acetate:hexanes) to give the diasteriomeric racemates as oils.

*Racemate A (1.03 gm):*

NMR ($CDCl_3$, TMS) 1.25 (overlapping t, 6H), 1.4—2.8 (m, 14H), 2.9—3.8 (m, 5H), 3.9—4.4 (m, + overlapping q, 5H), 7.1 + 7.3 (overlapping s, 9H).

*Racemate B (1.18 gm):*

NMR ($CDCl_3$, TMS) 1.1 (t, 3H), 1.25 (t, 3H), 1.5—3.7 (m, 19H), 3.8—4.3 (m, + overlapping q, 5H), 7.2 (s, 9H).

C. 1-(4-carboxybutyl)-3-(1-carboxy-3-phenyl-1-propyl)aminohomodihydrocarstyril

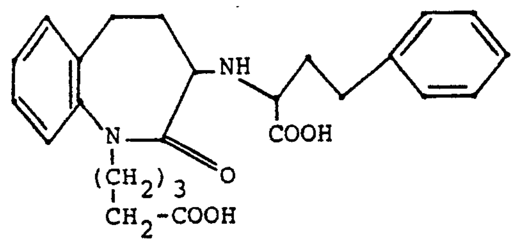

Q

*Racemate B:*

1 gm of diester racemate B was converted to the diacid by the procedure in Example 2.

*Diacid Racemate B (440 mg):*

NMR ($D_2O$, $ND_3$) 1.5—3.2 (m, 14M), 3.4—4.5 (m, 4H) 7.1 + 7.3 (overlapping s, 9H).

| | | | |
|---|---|---|---|
| El. An. Calc. for $C_{25}H_{30}N_2O_5$: | N, 6.39; | C, 68.48; | H, 6.90 |
| Found: | N, 6.45; | C, 68.22; | H, 6.87 |

Example 7

A. Ethoxycarbonylethyl-3-aminodihydrocarbostyril

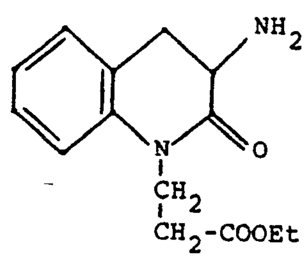

R

To a suspension of 3.38 gm NaH (50% oil dispersion washed 3 × hexanes) in 80 ml of THF at 0°C was added solid 7 gm (0.035 mol) of 3-aminodihydrocarbostyrilhydrochloride (T. J. McCord, Arch. of Biochem. & Biophys. *102* 48 (1963)) stirring at 0°C until the evolution of hydrogen had ceased at which time the

reaction mixture was warmed to room temperature and stirred a further 1 hour. To the stirred reaction mixture was added dropwise a solution of 4.75 gm of ethyl 3-iodopropionate in 20 ml of THF stirring a further 2 hours at room temperature at which time the reaction was quenched with 20 ml of saturated $NaHCO_3$. The reaction mixture was diluted with 20 ml of $H_2O$ and extracted 2 × 50 ml of 9:1 ethyl acetate:acetonitrile. The organic layers were combined, filtered through $MgSO_4$ and concentrated *in vacuo* to give 6.5 gm of 1-ethoxycarbonylethyl-3-aminodihydrocarbostyril.

NMR ($CDD_2OCl_3$, TMS) 1.15 (t, 3H), 2.4—3.0 (m, 5H), 3.1—3.7 (m, 1H), 3.8—4.2 (m, 3H), 6.8—7.2 (m, 4H).

B. 1-Ethoxycarbonylethyl-3-(1-carboethoxy-3-phenyl-1-propyl)aminodihydrocarbosltyril (racemate mixture)

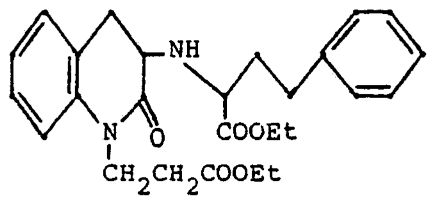

S

A solution of 4 gm (0.0153 mol) of 1-ethoxycarbonylethyl-3-aminodihydrocarbostyril, 12 gm of ethyl 4-phenyl-2-oxobutyrate and 0.875 ml of acetic acid in 30 ml of ethanol was stirred 1 hour at room temperature at which time was added dropwise over 8 hours a solution of 2.4 gm of $NaCNBH_3$ in 20 ml of absolute ethanol. After stirring a further 4 hours at room temperature the reaction mixture was concentrated *in vacuo*. It was then partitioned between water and ethyl acetate. The aqueous layer was extracted 2× with ethyl acetate and the combined organic layers filtered through $MgSO_4$ and concentrated at reduced pressure. The crude product was chromatographed (silica, 3:1 hexanes:ethyl acetate) to give two diasteriomeric racemates as oils.

*Racemate A (1.35 gm):*

TLC (silica, 7.3, hexanes:ethyl acetate) $R_f = 0.61$.

NMR ($CDCl_3$, TMS) 1.3 (t, 3H), 1.8—3.0 (m, 9H), 3.2—3.7 (m, 3H), 3.9—4.4 (m, 5H), 7.0—7.3 (m, 9H).

| | | | |
|---|---|---|---|
| El. An. Calc. for $C_{26}H_{32}N_2O_5$: | N, 6.19; | C, 69.00; | H, 7.13 |
| Found: | N, 6.01; | C, 68.81; | H, 7.08 |

*Racemate B (1.9 gm):*

TLC (silica, 2:1 hexanes:ethyl acetate) $R_f = 0.52$.

NMR ($CDCl_3$, TMS) 1.1 (t, 3H), 1.2 (t, 3H), 1.6—2.2 (m, 3H), 2.3—3.0 (m, 7H), 3.0—3.6 (m, 2H); 3.8—4.3 (m, 5H), 7.1 (bs, 9H).

| | | | |
|---|---|---|---|
| El. An. Calc. for $C_{26}H_{32}N_2O_5$: | N, 6.19; | C, 69.00; | H, 7.13 |
| Found: | N, 5.93; | C, 69.26; | H, 7.15 |

C. 1-Carboxyethyl-3-(1-carboxy-3-phenyl-1-propyl)aminodihydrocarbostyril

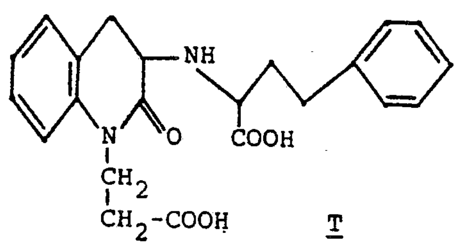

T

*Racemate B*

0.6 gm of diester racemate B was converted to the diacid by the procedure in Example 2.

*Diacid.Racemate B (410 mg):*

M.P. 198—200°C

NMR ($D_2O$) 1.8—2.4 (m, 2H);, 2.5—3.2 (m, 6H), 4.4 (bt, 2H) 7.3 + 7.4 (overlapping s, 9H).

| | | | |
|---|---|---|---|
| El. An. Calc. for $C_{22}H_{24}N_2O_5 \cdot 1H_2O$: | N, 6.76; | C, 63.70; | H, 6.27 |
| Found: | N, 6.66; | C, 63.75; | H, 6.04 |

Example 8

1-(3-Carboxypropyl)-3-(1-carboxy-3-phenyl-1-propyl)aminohexahydrobenzoazocine-2-one

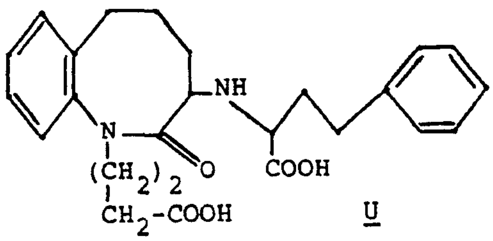

U

The title compound as a mixture of diasteriomeric racemates may be prepared by the sequence of reactions outlined in Examples 3 and 4 beginning with the alkylation of 3-azidohexahydrobenzoazocine-2-one (Example 3, Step C) with ethyl 4-iodobutyrate, followed by catalytic reduction to the free amine (Example 3, Step D). The free amine is then reductively alkylated with ethyl 4-phenyl-2-oxobutyrate and the diasteriomeric diester racemates separated by chromatography. (Example 3, Step E). The diester racemates are then hydrolyzed to the diacids (Example 4).

Example 9

1-(4-Carboxybutyl)-3-(1-carboxy-3-phenyl-1-propyl)aminohexahydrobenzoazocine-2-one

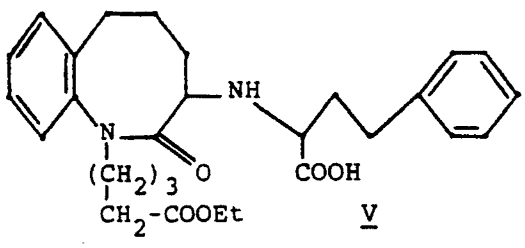

V

The title compound as a mixture of diasteriomeric racemates may be prepared from the sequence outlined in Example 8 beginning with the alkylation of 3-azidohexahydrobenzoazocine-2-one with ethyl 5-iodopentanoate.

Example 10

1-(3-Carboxypropyl)-3-(1-carboxy-3-phenyl-1-propyl)aminodihydrocarbostyril

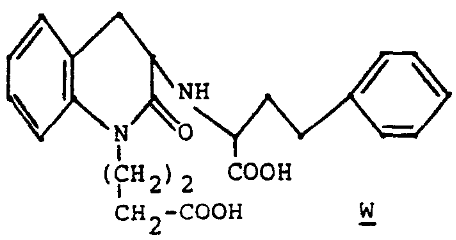

W

The title compound as a mixture of diasteriomeric racemates may be prepared by the synthetic sequence outlined in Example 7. The sequence would begin with the alkylation of 3-aminodihydrocarbostyril hydrochloride with ethyl 3-iodobutyrate followed by reductive alkylation with ethyl 4-phenyl-2-oxobutyrate. The diasteriomeric racemates are separated by chromatography and hydrolyzed to the diacids.

Example 11

1-(4-Carboxybutyl)-3-(1-carboxy-3-phenyl-1-propyl)aminodihydrocarbostyril

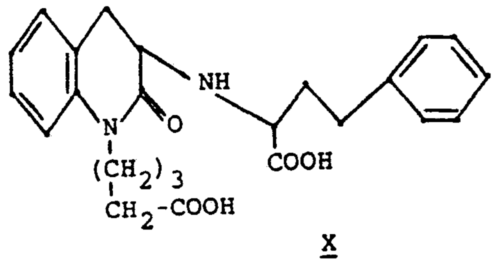

X

The title compound as a mixture of diasteriomeric racemates may be prepared by the sequence outlined in Example 10. The synthesis begins with the alkylation of 3-aminodihydrocarbostyril hydrochloride with ethyl 5-iodopentenoate.

Example 12

A. 1-Ethoxycarbonylethyl-3-(1-t-butoxycarbonyl-3-phenyl-1-propyl)aminohomodihydrocarbostyril (racemate mixture)

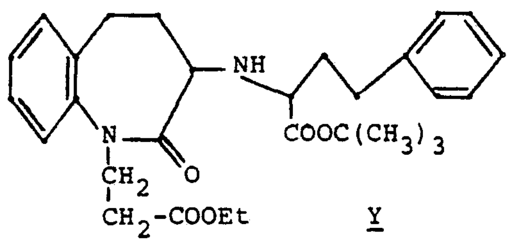

Y

A solution of 1 gm (0.0036 mol) of ethoxycarbonylethyl-3-aminohomodihydrocarbostyril, 2.95 gm of t-butyl 4-phenyl-2-oxobutyrate, and 0.32 gm of acetic acid in 7 ml of absolute ethanol was stirred 1 hour at room temperature. To the stirred solution was added dropwise over 16 hours a solution of 0.57 gm $NaCNBH_3$ in 6 ml of absolute ethanol. The reaction mixture was then concentrated at reduced pressure and partitioned between water and ethyl acetate. After extraction with ethyl acetate the combined organic layers were filtered through $MgSO_4$, concentrated *in vacuo* and chromatographed (silica, 7:3 hexane:ethyl acetate) to give the diasteriomeric racemates as oils.

*Racemate A (510 mg):*

NMR ($CDCl_3$, TMS) 1.2 (t, 3H), 1.5 (s, 9H), 1.6—3.4 (m, 13H), 3.6—4.8 (m, 4H), 7.1 + 7.2 (overlapping s, 9H).

TLC (silica, 2:1 hexanes:ethyl acetate) $R_f = 0.54$

*Racemate B (436 mg):*

NMR ($CDCl_3$, TMS) 1.2 (t, 3H), 1.3 (s, 9H), 1.8—3.4 (m, 13H) 3.6—4.8 (m, 4H), 7.2 (s, 9H).

TLC (silica, 7:3 hexanes:ethyl acetate) $R_f = 0.42$.

B. 1-Carboethoxyethyl-3-(1-carboxy-3-phenyl-1-propyl)aminohomodihydrocarbostyril

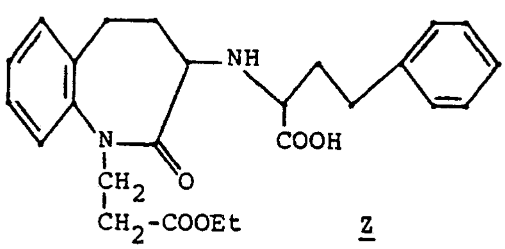

Z

*Racemate B:*

A solution of 0.43 gm of the diester racemate B in 5 ml of trifluoroacetic acid was stirred 3 hours at room temperature at which time the solution was concentrated *in vacuo* to give the monoacid ester.

*Monoacid Racemate B (300 mg):*
NMR ($CDCl_3$, TMS) 1.1 (t, 3H), 1.5—3.1 (m, 12H), 3.4—4.8 (m, 5H), 7.2 (bs, 9H).

**Claims**

1. A compound of the formula

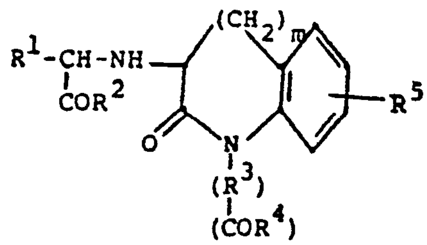

wherein
$R^1$ is alkyl or 1—10 carbon atoms which include branched, cyclic and unsaturated alkyl groups; substituted $C_{1-6}$alkyl wherein the substituent can be hydroxy, $C_{1-6}$alkylthio, amino, $C_{1-6}$alkylamino, di-$C_{1-6}$alkylamino, $C_{1-6}$alkanoylamino, and benzoylamino; a group having the formula $R^1_A(CH_2)_n$—Q—$(CH_2)_p$— wherein n is 0—2, p is 1—3, $R^1_A$ is phenyl, naphthyl, biphenyl or a 5- or 6-membered aromatic ring containing from one to three heteroatoms selected from the group consisting of nitrogen, oxygen and sulfur as well as any bicyclic group in which any of the above heterocyclic rings is fused to a benzene ring optionally substituted by $C_{1-6}$alkyl, halo, dihalo, amino, cyano, hydroxy, or $C_{1-6}$alkoxy, and Q is O, S, N—$R^1_B$, $CONR^1_C$, $NR^1_CCO$, or CH=CH wherein $R^1_B$ is hydrogen, $C_{1-6}$alkyl, phenyl, naphthyl, or biphenyl attached through a straight or branched chain hydrocarbon of from 1 to 6 carbon atoms, $C_{1-6}$alkanoyl, or benzoyl and $R^1_C$ is hydrogen or $C_{1-6}$alkyl; $C_{1-6}$alkyl substituted by phenyl, naphthyl, biphenyl or a 5- or 6-membered aromatic ring containing from one to three heteroatoms selected from the group consisting of nitrogen, oxygen and sulfur as well as any bicyclic group in which any of the above heterocyclic rings is fused to a benzene ring wherein the $C_{1-6}$alkyl portion can be substituted by amino, $C_{1-6}$alkanoylamino, benzoylamino or hydroxy and the aromatic ring portions can be substituted by $C_{1-6}$alkyl, halo, dihalo, amino, cyano, hydroxy, $C_{1-6}$alkoxy, amino-$C_{1-6}$alkyl, or hydroxy-$C_{1-6}$alkyl;
$R^2$ and $R^4$ are hydroxy; $C_{1-6}$alkoxy; phenyloxy, naphthyloxy, biphenyloxy; or $C_{1-6}$alkoxy substituted by phenyloxy, naphthyloxy, biphenyloxy;
m is 1 to 4;

$R^3$ is 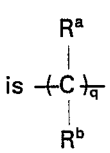

where
q is 2 or 3; provided that where q = 3, the carbon chain may be unsaturated between carbons 2—3; the $R^a$'s and $R^b$'s are hydrogen; or $C_{1-6}$alkyl; and the $R^a$'s are additionally phenyl-$C_{1-6}$alkyl; amino-$C_{1-6}$alkyl; or $C_{1-6}$alkylthio-$C_{1-6}$alkyl; and
$R^5$ is hydrogen; or hydroxy;
and a pharmaceutically acceptable salt thereof.

2. A compound according to Claim 1 selected from the group consisting of:
1-ethoxycarbonylethyl-3-(1-carboethoxy-3-phenyl-1-propyl)aminohomodihydrocarbostyril;
1-carboxyethyl-3-(1-carboxy-3-phenyl-1-propyl)aminohomodihydrocarbostyril;
1-carboxypropyl)-3-(1-carboxy-3-phenyl-1-propyl)aminohomodihydrocarbostyril, and ethyl diester;
1-(4-carboxybutyl)-3-(1-carboxy-3-phenyl-1-propyl)aminohomodihydrocarbostyril, and ethyl diester;
1-ethoxycarbonylethyl-3-(1-carboxy-3-phenyl-1-propyl)aminohomodihydrocarbostyril.

3. A compound according to Claim 1 selected from the group consisting of:
1-carboxethyl-3-(1-carboxy-3-phenyl-1-propyl)aminodihydrocarbostyril, and ethyl diester;
1-(3-carboxypropyl)-3-(1-carboxy-3-phenyl-1-propyl)aminodihydrocarbostyril, and ethyl diester;
1-(4-carboxybutyl)-3-(1-carboxy-3-phenyl-1-propyl)aminodihydrocarbostyril, and ethyl diester.

4. A compound according to Claim 1 selected from the group consisting of:
1-ethoxycarbonylethyl-3-(1-carboethoxy-3-phenyl-1-propyl)aminohexahydrobenzoazocine-2-one;
1-carboxyethyl-3-(1-carboxy-3-phenyl-1-propyl)aminohexahydrobenzoazocine-2-one and ethyl diester;
1-(3-carboxypropyl)-3-(1-carboxy-3-phenyl-1-propyl)aminohexahydrobenzoazocine-2-one, and ethyl diester;
1-(4-carboxybutyl)-3-(1-carboxy-3-phenyl-1-propyl)aminohexahydrobenzoazocine-2-one, and ethyl diester;

5. A pharmaceutical composition useful in treating hypertension comprising a pharmaceutically acceptable carrier and an antihypertensively effective amount of a compound of Claim 1.

**Patentansprüche**

1. Eine Verbindung der Formel

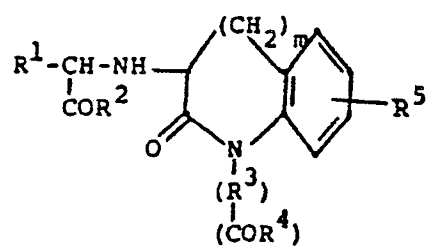

worin bedeuten

$R^1$ Alkyl mit bis 10 Kohlenstoffatomen, worin verzweigte, cyclische und ungesättigte Alkylreste eingeschlossen sind;

substituiertes $C_{1-6}$Alkyl worin der Substituent Hydroxy, $C_{1-6}$Alkylthio, Amino, $C_{1-6}$Alkylamino, Di-$C_{1-6}$alkylamino, $C_{1-6}$Alkanoylamino, und Benzoylamino sein kann;

eine Gruppe mit der Formel $R^1_A(CH_2)_n$—Q—$(CH_2)_p$— worin n 0—2, p 1—3 ist, $R^1_A$ Phenyl, Naphthyl, Biphenyl, einen 5- oder 6-gliedrigen, aromatischen Ring, der 1—3 Heteroatome, ausgewählt aus der Gruppe bestehend aus Stickstoff, Sauerstoff und Schwefel, enthält, sowie eine bicyclische Gruppe, in welcher jeder der obigen heterocyclischen Ringe mit einem Benzolring verschmolzen ist, bedeutet, gegebenenfalls substituiert durch $C_{1-6}$Alkyl, Halogen, Dihalogen, Amino, Cyano, Hydroxy oder $C_{1-6}$Alkoxy, und Q Sauerstoff, Schwefel, N—$R^1_B$, $CONR^1_C$, $NR^1_CCO$ oder CH=CH ist, worin $R^1_B$ Wasserstoff, $C_{1-6}$Alkyl, Phenyl, Naphthyl oder Biphenyl, verknüpft durch eine gerade oder verzweigte Kohlenwasserstoffkette von 1 bis 6 Kohlenstoffatomen, $C_{1-6}$Alkanoyl oder Benzoyl ist und worin $R^1_C$ Wasserstoff, $C_{1-6}$Alkyl oder substituiertes $C_{1-6}$Alkyl bedeutet, worin der Substituent Phenyl, Naphthyl, Biphenyl, einen 5- oder 6-gliedrigen aromatischen Ring, enthaltend 1—3 Heteroatome, ausgewählt aus der Gruppe bestehend aus Stickstoff, Sauerstoff und Schwefel, sowie eine bicyclische Gruppe, in welcher jeder der obigen heterocyclischen Ringe mit einem Benzolring verschmolzen ist, bedeutet, worin der $C_{1-6}$Alkylteil durch Amino, $C_{1-6}$Alkanoylamino, Benzoylamino oder Hydroxy substituiert sein kann und worin die aromatischen Ringteile durch $C_{1-6}$Alkyl, Halogen, Dihalogen, Amino, Cyano, Hydroxy, $C_{1-6}$Alkoxy, Amino-$C_{1-6}$alkyl oder Hydroxy-$C_{1-6}$Alkyl substituiert sein können;

$R^2$ und $R^4$ Hydroxy, $C_{1-6}$Alkoxy, Phenyloxy, Naphthyloxy, Biphenyloxy oder substituiertes $C_{1-6}$Alkoxy, worin der Substituent Phenyloxy, Naphthyloxy oder Biphenyloxy ist;

m 1 bis 4;

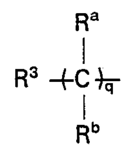

worin q 2 oder 3 ist, wobei, wenn q = 3 ist, die Kohlenstoffkette zwischen dem zweiten und dritten Kohlenstoffatom ungesättigt sein kann; und worin $R^a$ und $R^b$ Wasserstoff oder $C_{1-6}$Alkyl sind und $R^a$ zusätzlich Phenyl-$C_{1-6}$alkyl, Amino-$C_{1-6}$alkyl oder $C_{1-6}$Alkylthio-$C_{1-6}$alkyl bedeutet; und

$R^5$ Wasserstoff oder Hydroxy;

sowie ein pharmazeutisch annehmbares Salz hiervon.

2. Verbindung gemäss Anspruch 1, ausgewählt aus der Gruppe bestehend aus:

1-Ethoxycarbonylethyl-3-(1-carboethoxy-3-phenyl-1-propyl)aminohomodihydrocarbostyril;

1-Carboxyethyl-3-(1-carboxy-3-phenyl-1-propyl)aminohomodihydrocarbostyril;

1-(3-Carboxypropyl)-3-(1-carboxy-3-phenyl-1-propyl)aminohomodihydrocarbostyril und der Diethyl ester;

1-(4-Carboxybutyl)-3-(1-carboxy-3-phenyl-1-propyl)aminohomodihydrocarbostyril, und der Diethylester;

1-Ethoxycarbonylethyl-3-(1-carboxy-3-phenyl-1-propyl)aminohomodihydrocarbostyril.

3. Eine Verbindung gemäss Anspruch 1, ausgewählt aus der Gruppe bestehend aus:

1-Carboxethyl-3-(1-carboxy-3-phenyl-1-propyl)aminodihydrocarbostyril und der Diethylester;

1-(3-Carboxypropyl)-3-(1-carboxy-3-phenyl-1-propyl)aminodihydrocarbostyril und der Diethylester;

1-(4-Carboxybutyl)-3-(1-carboxy-3-phenyl-1-propyl)aminodihydrocarbostyril und der Diethylester.

4. Eine Verbindung gemäss Anspruch 1, ausgewählt aus der Gruppe bestehend aus:

1-Ethoxycarbonylethyl-3-(1-carboethoxy-3-phenyl-1-propyl)aminohexahydrobenzoazocin-2-on;

1-Carboxyethyl-3-(1-carboxy-3-phenyl-1-propyl)aminohexahydrobenzoazocin-2-on und der Diethylester;

1-(3-Carboxypropyl)-3-(1-carboxy-3-phenyl-1-propyl)aminohexahydrobenzoazocin-2-on und der Diethylester;

1-(4-Carboxybutyl)-3-(1-carboxy-3-phenyl-1-propyl)aminohexahydrobenzoazocin-2-on und der Diethylester.

5. A Pharmazeutische Zubereitung, brauchbar für die Behandlung von Hypertonie, enthaltend, einen pharmazeutisch annehmbaren Träger und eine antihypertonisch wirksame Menge einer Verbindung gemäss Patentanspruch 1.

**Revendications**

1. Un composé de formule

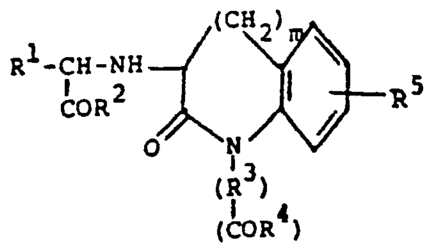

dans laquelle

$R^1$ est un alkyle de 1 à 10 atomes de carbone qui comprend les groupes alkyles ramifiés, cycliques et insaturés; un alkyle en $C_{1-6}$ substitué dont le substituant peut être hydroxy, alkylthio en $C_{1-6}$, amino, alkylamino en $C_{1-6}$, di(alkyl en $C_{1-6}$)amino, alcanoylamino en $C_{1-6}$ et benzoylamino; un group de formule $R^1_A(CH_2)_n—Q—(CH_2)_p—$ dans laquelle n est 0—2, p est 1—3, $R^1_A$ est un phényle, naphtyle ou biphényle, ou un cycle aromatique à 5 ou 6 chaînons contenant de 1 à 3 hétéro-atomes choisis dans le groupe constitué par l'azote, l'oxygène et le soufre, ainsi que tout groupe bicyclique dans lequel l'un quelconque des hétérocycles ci-dessus est condensé à un noyau benzène éventuellement substitué par un alkyle en $C_{1-6}$, un halogéno, un dihalogéno, un amino, un cyano, un hydroxy ou un alcoxy en $C_{1-6}$, et Q est O, S, $N—R^1_B$, $CONR^1_C$, $NR^1_CCO$ ou CH=CH où $R^1_B$ est un hydrogène, un alkyle en $C_{1-6}$, un phényle, un naphtyle ou un biphényle fixé par l'intermédiaire d'un hydrocarbure à chaîne droite ou ramifiée de 1 à 6 atomes de carbone, un alcanoyle en $C_{1-6}$ ou un benzoyle et $R^1_C$ est un hydrogène ou un alkyle en $C_{1-6}$;

un alkyle en $C_{1-6}$ substitué par un phényle, un naphtyle, un biphényle ou un cycle aromatique à 5 ou 6 chaînons contenant de 1 à 3 hétéro-atomes choisis dans le groupe constitué par l'azote, l'oxygène et le soufre, ainsi que tout groupe bicyclique dans lequel un des hétérocycles ci-dessus est condensé à un noyau benzène où la portion alkyle en $C_{1-6}$ peut être substitué par un amino, un alcanoylamino en $C_{1-6}$, un benzoylamino ou un hydroxy et les portions de cycle aromatique peuvent être substituées par un alkyle en $C_{1-6}$, un halogéno, un dihalogéno, un amino, un cyano, un hydroxy, un alcoxy en $C_{1-6}$, un aminoalkyle en $C_{1-6}$ ou un hydroxyalkyle en $C_{1-6}$;

$R^2$ et $R^4$ sont un hydroxy; un alcoxy en $C_{1-6}$; un phénoxy, un naphtyloxy, un biphénoxy ou un alcoxy en $C_{1-6}$ substitué par un phénoxy, un naphtyloxy ou un biphénoxy;

m est 1 à 4;

$$R^3 \text{ est } -\!\left(\overset{\displaystyle R^a}{\underset{\displaystyle R^b}{C}}\right)_{\!q}\!-$$

où q est 2 ou 3; sous réserve que, lorsque q = 3, la chaîne carbonée peut être insaturée entre les carbones 2—3;

les $R^a$ et $R^b$ sont un hydrogène; ou un alkyle en $C_{1-6}$; et les $R^a$ sont de plus un phényl-alkyle en $C_{1-6}$; un aminoalkyle en $C_{1-6}$; ou un alkyl(en $C_{1-6}$)thioalkyle en $C_{1-6}$; et

$R^5$ est un hydrogène ou un hydroxy;

et un sel pharmaceutiquement acceptable de ceux-ci.

2. Un composé selon la revendication 1 choisi dans le groupe constitué par:

1-éthoxycarbonyléthyl-3-(1-carboéthoxy-3-phényl-1-propyl)aminohomodihydrocarbostyryle;

1-carboxyéthyl-3-(1-carboéthoxy-3-phényl-1-propyl)aminohomodihydrocarbostyryle;

1-(3-carboxypropyl)-3-(1-carboxy-3-phényl-1-propyl)aminohomodihydrocarbostyryle, et diester éthylique;

1-(4-carboxybutyl)-(1-carboxy-3-phényl-1-propyl)aminohomodihydrocarbostyryle, et diester éthylique;

1-éthoxycarbonyléthyl-(1-carboxy-3-phényl-1-propyl)aminohomodihydrocarbostyryle.

3. Un composé selon la revendication 1 choisi dans le groupe constitué par:

1-carboxyéthyl-3-(1-carboxy-3-phényl-1-propyl) aminodihydrocarbostyryle, et diester éthylique;

1-(3-carboxypropyl)--3-(1-carboxy-3-phényl-1-propyl) aminodihydrocarbostyryle, et diester éthylique;

1-(4-carboxybutyl)--3-(1-carboxy-3-phényl-1-propyl) aminodihydrocarbostyryle, et diester éthylique.

4. Un composé selon la revendication 1 choisi dans le groupe constitué par:

1-éthoxycarbonyléthyl-3-(1-carboéthoxy-3-phényl-1-propyl)aminohexahydrobenzoazocine-2-one;

1-carboxyéthyl-3-(1-carboxy-3-phényl-1-propyl)aminohexahydrobenzoazocine-2-one, et diester éthylique;

1-(3-carboxypropyl)-3-(1-carboxy-3-phényl-1-propyl)aminohexahydrobenzoazocine-2-one, et diester éthylique;

1-(4-carboxybutyl)-3-(1-carboxy-3-phényl-1-propyl)aminohexahydrobenzoazocine-2-one, et diester éthylique.

5. Une composition pharmaceutique utile dans le traitement de l'hypertension comprenant un support pharmaceutiquement acceptable et une quantité antihypertensive efficace d'un composé de la revendication 1.